# EUROPEAN PATENT SPECIFICATION

(11) **EP 2 134 750 B1**
(45) Date of publication and mention of the grant of the patent: **06.04.2016**
(21) Application number: 08735191.2
(22) Date of filing: 11.04.2008
(51) Int. Cl.: A61K 39/00

(54) **IG-P CONSENSUS GENE VACCINATION PROTECTS FROM ANTIBODY-DEPENDENT IMMUNE PATHOLOGY IN AUTOIMMUNE DISEASE**
IG-P-KONSENSGEN-IMPFSTOFF ZUM SCHUTZ VOR ANTIKÖRPERVERMITTELTER IMMUNPATHOLOGIE BEI AUTOIMMUNERKRANKUNGEN
VACCINATION UTILISANT DES GÈNES CONSENSUS IG-P PERMETTANT D'OBTENIR UNE PROTECTION CONTRE UNE PATHOLOGIE IMMUNITAIRE DÉPENDANT D'ANTICORPS ASSOCIÉS À UNE MALADIE AUTO-IMMUNE

(30) Priority: 11.04.2007 US 911267 P
(43) Date of publication of application: 23.12.2009
(73) Proprietor: The Regents of the University of California, Oakland, CA 94607 (US); Università Degli Studi Di Genova, 16124 Genova (IT)
(72) Inventor: LA CAVA, Antonio, Santa Monica, California 90403 (US); HAHN, Bevra, H., Encino, California 91436 (US); FILACI, Gilberto, I-16147 Genova (IT); FERRERA, Francesca, I-16134 Genova (IT); RIZZI, Marta, I- 16128 Genova (IT); INDIVERI, Francesco, I-16131 Genova (IT)
(74) Representative: Weickmann & Weickmann
(86) International application number: PCT/EP2008/002902
(87) International publication number: WO 2008/125307

(56) References cited:
- TISCH ET AL: "Antigen-specific mediated suppression of .beta. cell autoimmunity by plasmid DNA vaccination" JOURNAL OF IMMUNOLOGY, THE WILLIAMS AND WILKINS CO. BALTIMORE, vol. 166, no. 3, 1 February 2001 (2001-02-01), pages 2122-2132, XP002295858 ISSN: 0022-1767
- HAHN BEVRA H ET AL: "Treatment with a consensus peptide based on amino acid sequences in autoantibodies prevents T cell activation by autoantigens and delays disease onset in murine lupus", ARTHRITIS & RHEUMATISM, JOHN WILEY & SONS, INC, US, vol. 44, no. 2, 1 February 2001 (2001-02-01), pages 432-441, XP009172309, ISSN: 0004-3591 [retrieved on 2001-02-28]

## Description

### TECHNICAL FIELD

The invention relates to methods and compositions useful for treating autoimmune diseases and disorders. In one aspect, the invention provides genetic constructs and polypeptides and methods for treating systemic lupus erythematosus (SLE).

### BACKGROUND

The presence of hypergammaglobulinemia often associates with chronic inflammatory conditions and is commonly observed in systemic lupus erythematosus (SLE), an autoimmune disease characterized by multiple antibodies (Ab) to self-antigens that can form immunocomplexes depositing in the kidney, a process leading to loss of renal function.

(NZB x NZW)F₁ (NZB/W F₁) mice spontaneously develop a systemic autoimmune disease that closely resembles human SLE. These animals develop serum auto-Ab to several self-Ag including double stranded (ds)DNA, chromatin and histones, and die of renal failure secondary to deposition of pathogenic Ab and immune complexes in the kidney glomeruli. Although B cells are crucial for the development of SLE and genetic deficiency of these lymphocytes can protect from lupus, T cells are equally important in the pathogenesis of the disease. In particular, T helper (Th) cells in SLE can recognize T-cell determinants within idiotypes of auto-Ab and provide help to B cells for the production of auto-Ab. Nonetheless, the elevated levels of polyclonal IgG in SLE represents a major pathogenetic component of the disease that contributes highly both to its morbidity and mortality.

### SUMMARY

An increased production of polyclonal IgG (hypergammaglobulinemia) and a perturbation of humoral immune responses are important characteristics of systemic lupus erythematosus (SLE). Similarly to humans, female (NZB x NZW)F₁ (NZB/W F₁) lupus-prone mice have increased serum levels of IgG that can form immunocomplexes when reactive to self-antigen. Since those immunocomplexes can deposit in the kidney and cause glomerulonephritis - a major cause of mortality in SLE - a reduction of IgG production would likely benefit the prognosis of SLE. The invention demonstrates that somatic B-cell transfer of a minigene that encodes a consensus sequence of T-cell determinants in murine IgG can inhibit sustained elevated production of IgG NZB/W F₁ mice, with resulting protection from accelerated renal disease and subsequent increased survival of the animals. The mechanisms involved in the protection from hypergammaglobulinemia include an expansion of TGFbeta-producing CD8⁺CD28⁻ T cells that suppress antigen-specific stimulation of CD4⁺ T cells in a cell-contact independent manner. Significantly, the adoptive transfer of CD8⁺CD28⁻ T cells from minigene-protected mice into NZB/W F₁ mice with hypergammaglobulinemia also protects from development of renal disease. These data indicate the possibility of minigene-based induction of immunoregulatory circuits that can delay development of murine lupus nephritis by suppressing hypergammaglobulinemia.

The invention demonstrates that hypergammaglobulinemia and subsequent accelerated kidney disease can be suppressed in an animal model of SLE (e.g., NZB/W F₁ mice) by Ig minigene-induced CD8⁺ T cells that make CD4⁺ T cells hyporesponsive to antigenic stimulation, thus causing inhibition of renal disease and subsequent increased survival of the mice. The subject-matter of the present invention is defined by the attached claims. In particular, the present invention relates to a polynucleotide encoding a fusion polypeptide, wherein the fusion polypeptide comprises a self antigen operably linked to an Fc region of an antibody, wherein the self antigen is pCons SEQ ID NO: 2. Further, the invention relates to an expression vector comprising said polynucleotide. In a further aspect, the invention is directed to said polynucleotide for use in prevention or treatment of systemic lupus erythematosus by inducing tolerogenic immunity in a subject, wherein the polynucleotide is for expression in the subject. Moreover, the invention refers to a fusion polypeptide as defined in the present claims and to a pharmaceutical composition comprising said fusion polypeptide. Finally, the invention relates to said fusion polypeptide for use in the treatment of an autoimmune disorder for repressing an immune response to a self antigen, in particular to a self antigen comprising SEQ ID NO: 2, wherein the autoimmune disorder is systemic lupus erythematosus.

### BRIEF DESCRIPTION OF THE DRAWINGS

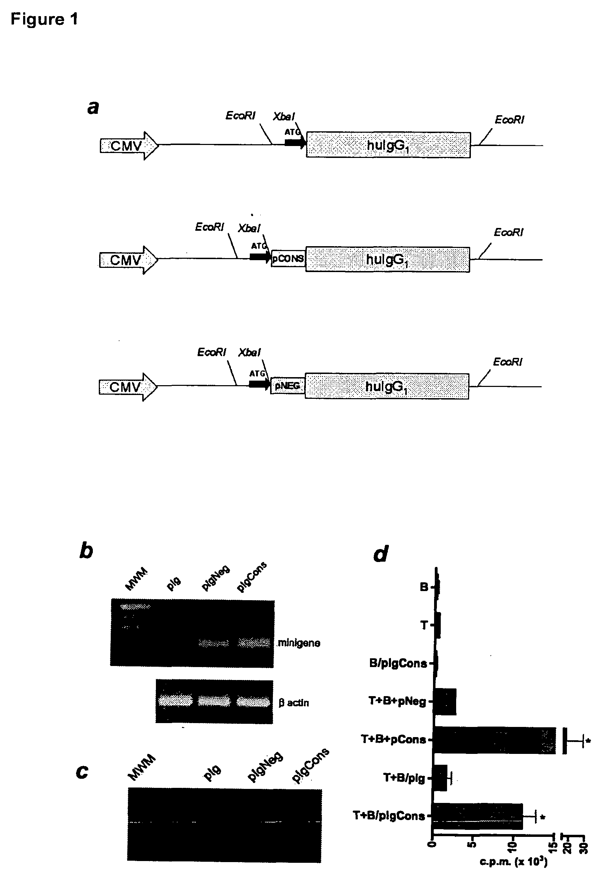

Figure 1. Minigene maps, transcripts and gene products. a. Schematic representation of constructs encoding hIgG₁ (Ig alone, pIg [top]; in combination with pCons, pIgCons [middle]; or in combination with pNeg, pIgNeg [bottom]). b. RT-PCR on RNA extracted from COS-7 cells transfected with the different minigenes (pIg, pIgCons, pIgNeg). The expected molecular size is marked on the left as "minigene". MWM, molecular weight marker. c. Western blot of fusion proteins of expected molecular weight on lysates of COS-7 cells transfected with the different minigenes. MWM, molecular weight marker. d. Proliferative responses of a pCons-specific T cell line (T) derived from mice immunized with pCons to B cells transfected with pIg (B/pIg) or pIgCons (B/plgCons); P<0.004. Specificity is indicated by lack of proliferation of B cells transfected with pIgCons when cultured alone (B/plgCons) and by optimal proliferation of the T cell line when co-cultured with B cells and pCons peptide but not when co-cultured with pNeg peptide. Representative of six experiments.

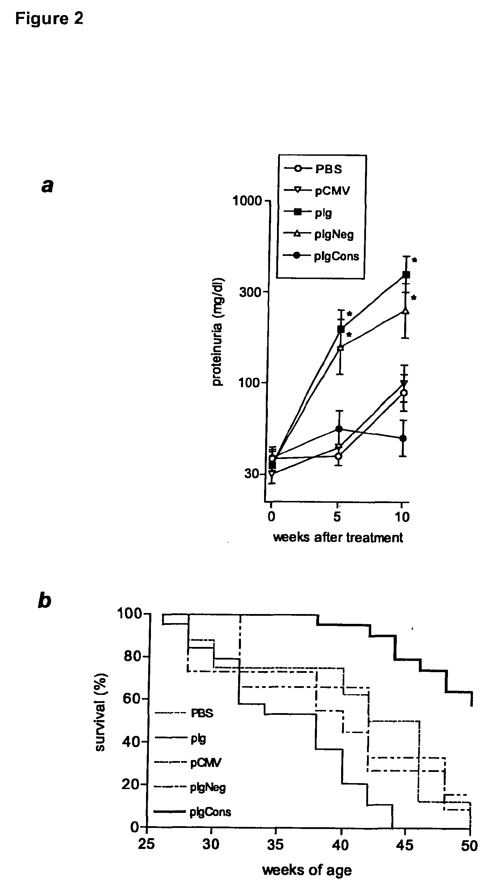

Figure 2. Treatment of NZB/W F₁ mice with pIgCons associates with delayed development of proteinuria and increased survival of treated animals. Each mouse received 6x10⁵ B cells transfected with the relative minigene as described in the Materials and Methods. The PBS control group only received PBS. a. Proteinuria five weeks after treatment, pIgCons vs pIg or pIgNeg, P<0.01. Ten weeks after treatment, pIgCons vs pIg or pIgNeg, P<0.0001 and P<0.0002, respectively. b. Mice were monitored for survival until 50 weeks after transfer of B cells transfected with pIg, pIgCons, pIgNeg, or pCMV plasmids. A control group of mice received only PBS. P<0.004 by Kaplan Meyer analysis.

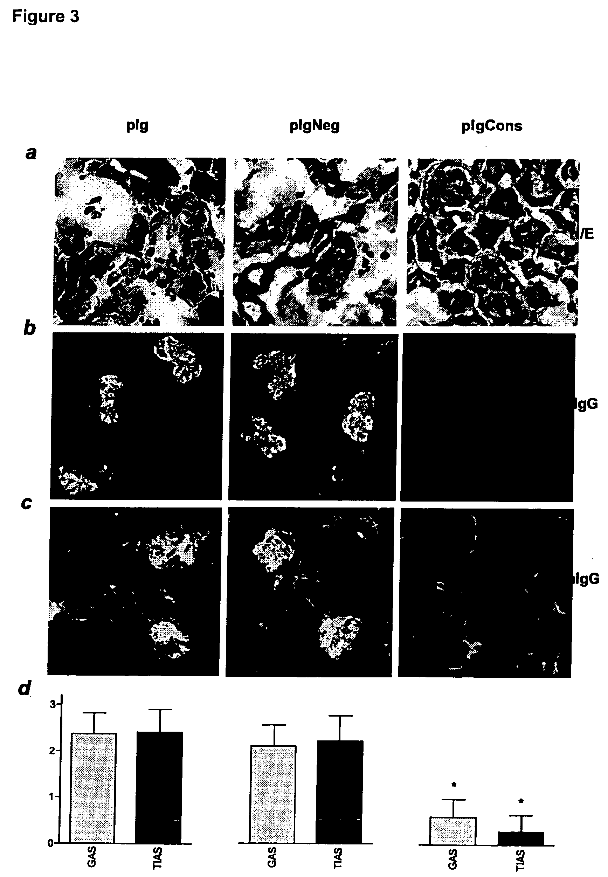

Figure 3. Histology of the kidneys of the mice used in the study. a. Hematoxylin-eosin staining shows that mice treated with pIgCons have reduced glomerular involvement and preserved tissue architecture compared to mice treated with pIg or pIgNeg. b-c. Immunofluorescence staining indicates increased hIgG (b) and mIgG (c) precipitation in the glomeruli of pIg and pIgNeg treated mice as compared to mice treated with pIgCons. Magnification: 200X. d. Cumulative glomerular activity score (GAS) and tubulointerstitial activity score (TIAS) of kidneys from mice treated with pIg (left), pIgNeg (middle), and pIgCons (right). P<0.0001 for both GAS and TIAS.

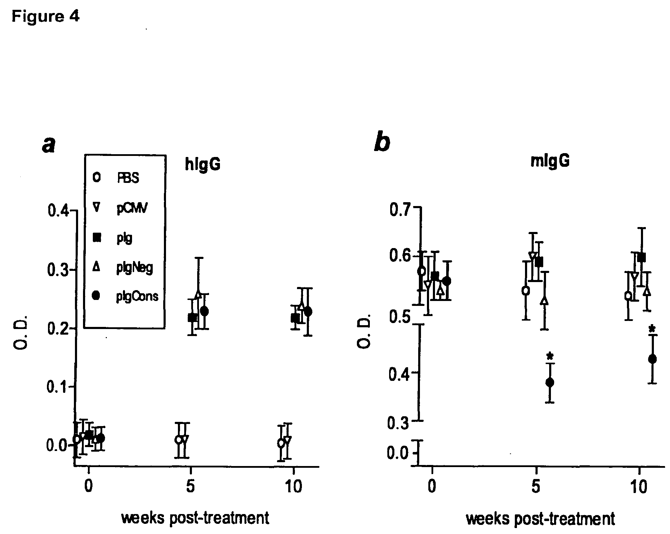

Figure 4. Anti-Ig responses after minigene vaccination. Mean ± SD of anti-human IgG (a) and anti-mouse IgG (b) responses in treated mice and controls (n = 6 to 12 per group) at 5 and 10 weeks after treatment. P<0.0001 at both 5 and 10 weeks.

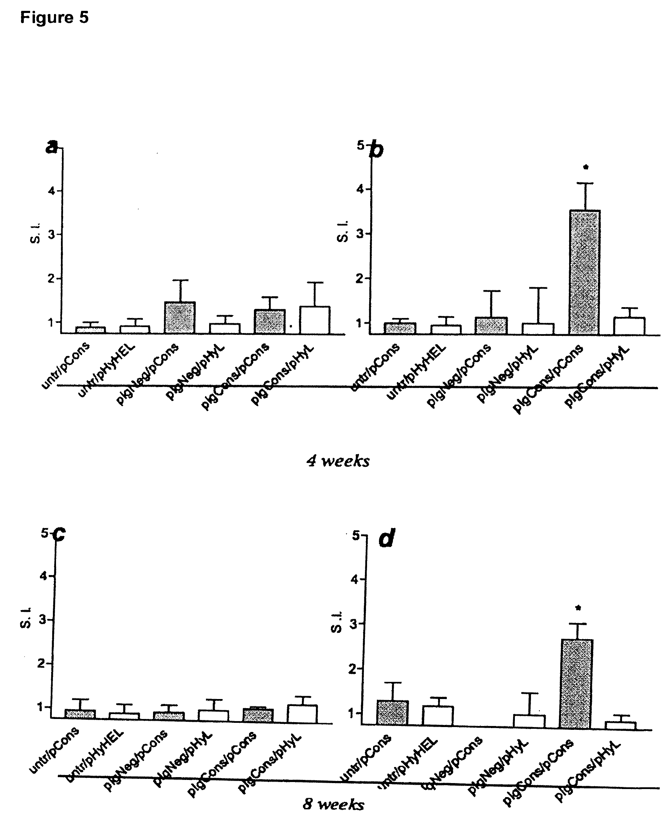

Figure 5. T cell responses to minigene vaccination. Ag-specific T cell responses were measured at 4 (a, b) and 8 (c, d) weeks after treatment. Mean (±SD) stimulation index is indicated on the y axis (4-9 mice per group). Background cpm: 0.5-2.0 x 10³. a and c, proliferation in the presence of peptides (x axis) only. b and d, proliferation in the presence of peptides (x axis) plus IL-2. P<0.07 at 4 weeks; P<0.05 at 8 weeks.

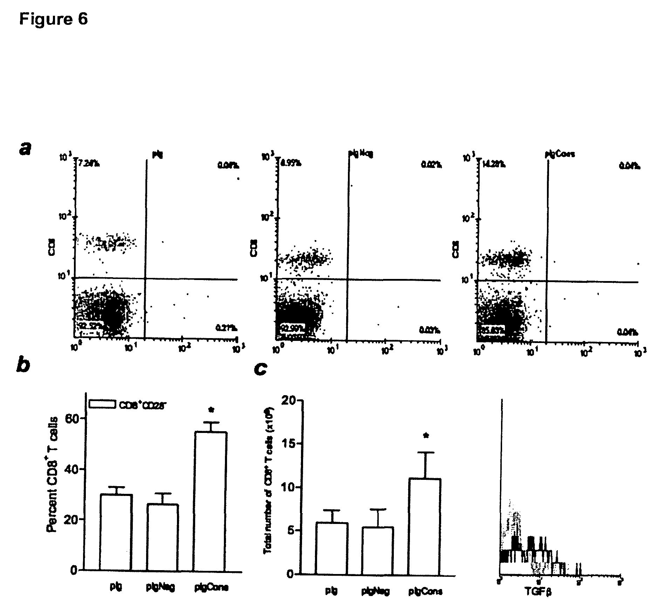

Figure 6. Flow cytometry analysis on peripheral mononuclear cells two weeks after minigene vaccination. a. Surface expression of CD8 on CD3⁺ T cells from mice treated with pIg (left), pIgNeg (center), and pIgCons (right) indicates an expansion of CD8⁺ cells in pIgCons mice as compared to plg- and pIgNeg-treated mice. b-c. Within the (gated) CD8⁺ T cell compartment, CD8⁺CD28⁻ cells expand in pIgCons mice but not in control mice; P<0.005 (b), P<0.001 (c). d. Staining for intracellular TGF-beta in gated CD8⁺CD28⁻ lymphocytes from pIgCons-treated mice (black) and from pIgNeg-treated mice (gray) indicates expression of this cytokine in T cells of the pIgCons group but not in the pIgNeg group of mice. Representative of duplicate experiments on individual mice (n=5/group).

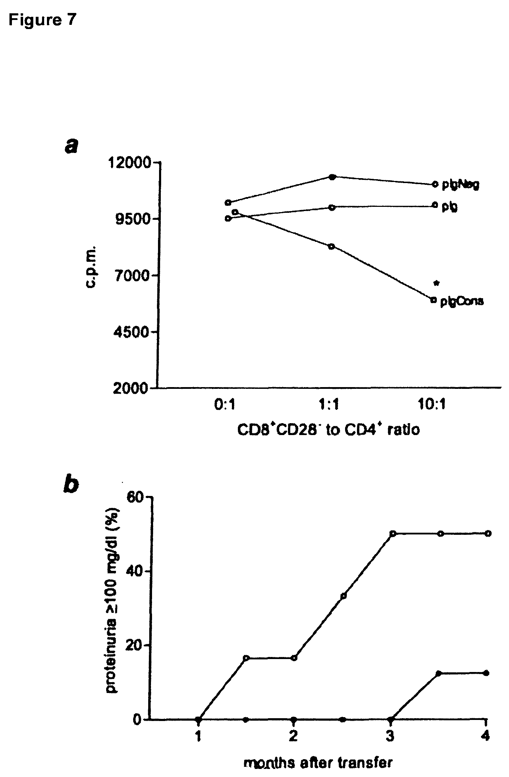

Figure 7. In vitro and in vivo activity of CD8⁺CD28⁻ lymphocytes of pIgCons-treated mice. a. CD8⁺CD28⁻ cells suppress in vitro the proliferation of CD4⁺ T cells (scalar doses of effector to target ratio); P<0.02 vs pIg or pIgNeg; not significant at 1:1 ratio. b. In vivo transfer of purified CD8⁺CD28⁻ T cells from pIgCons-treated mice delays proteinuria in mice with hypergammaglobulinemia. 1x10⁷ CD8⁺CD28⁻ T cells from mice treated with pIgCons (●) (n = 6) or pIgNeg (o) (n = 8) were transferred into female NZB/ W F₁ mice with serum IgG >10 mg/ml and recipients monitored every other week for development of proteinuria (≥100 mg/dl). P<0.001 by Kaplan Meyer analysis.

### DETAILED DESCRIPTION

The exemplary descriptions provided herein are exemplary and explanatory only and are not restrictive of the invention, as claimed. Moreover, the invention is not limited to the particular embodiments described, as such may, of course, vary. Further, the terminology used to describe particular embodiments is not intended to be limiting.

With respect to ranges of values, the invention encompasses each intervening value between the upper and lower limits of the range to at least a tenth of the lower limit's unit, unless the context clearly indicates otherwise. Further, the invention encompasses any other stated intervening values. Moreover, the invention also encompasses ranges excluding either or both of the upper and lower limits of the range, unless specifically excluded from the stated range.

Unless defined otherwise, the meanings of all technical and scientific terms used herein are those commonly understood by one of ordinary skill in the art to which this invention belongs. One of ordinary skill in the art will also appreciate that any methods and materials similar or equivalent to those described herein can also be used to practice or test the invention.

It must be noted that, as used herein and in the appended claims, the singular forms "a," "or," and "the" include plural referents unless the context clearly dictates otherwise. Thus, for example, reference to "a subject polypeptide" includes a plurality of such polypeptides and reference to "the agent" includes reference to one or more agents and equivalents thereof known to those skilled in the art, and so forth.

"CD8+ T cells" represent a class of T lymphocytes characterized by the possession of the CD8 cell surface marker. CD8+ T cells are MHC Class I-restricted "CTLs" or "suppressor T cells."

"CD4+ T cells" represent a class of T lymphocytes characterized by the possession of the CD4 cell surface marker. CD4+ T cells are MHC Class II-restricted T lymphocytes. There are two types of CD4+ T cells referred to as type 1 or type 2 "helper T cells."

An immune response is generated to an antigen through the interaction of the antigen with the cells of the immune system. The resultant immune response may be broadly distinguished into humoral or cell mediated immune responses (traditionally characterized by antibody and cellular effector mechanisms of protection, respectively). These categories of response have been termed Th1-type responses (cell-mediated response), and Th2-type immune responses (humoral response). Th1-type immune responses may be characterized by the generation of antigen-specific, haplotype-restricted CTLs, and natural killer cell responses. In mice, Th1-type responses are often characterized by the generation of antibodies of the IgG2a subtype, while in the human these correspond to IgG1 type antibodies. Th2-type immune responses are characterized by the generation of a broad range of immunoglobulin isotypes including in mice IgG1, IgA, and IgM.

A driving force behind the development of these two types of immune responses is cytokines, a number of identified protein messengers which serve to help the cells of the immune system and steer the eventual immune response to either a Th1 or Th2 response. Thus, high levels of Th1-type cytokines tend to favor the induction of cell mediated immune responses to the given antigen, while high levels of Th2-type cytokines tend to favor the induction of humoral immune responses to the antigen. It is important to remember that the distinction of Th1 and Th2-type immune responses is not absolute. Traditionally, Th1-type responses are associated with the production of the INF-γ and IL-2 cytokines by T-lymphocytes. Other cytokines often directly associated with the induction of Th1-type immune responses are not produced by T-cells, such as IL-12. In contrast, Th2-type responses are associated with the secretion of IL-4, IL-5, IL-6, IL-10 and tumor necrosis factor-β (TNF-β).

A difference between B cells and T cells is how the B- and T-cell recognize antigen. B cells recognize antigen in its native form. For example, they recognize antigen in the blood or lymph using membrane bound antigen recognition domains comprising bound-immunoglobulin. T cells, such as helper T-cells, recognize antigen in a processed form, as a peptide fragment presented by an antigen presenting cell's MHC molecule to the T cell receptor.

when a B cell recognizes an antigen, the B cell ingests through a process of endocytosis the antigen in combination with the immunoglobulin domain that recognized the antigen. The B cell then processes the antigen and attaches parts of the antigen to an MHC protein. This complex is moved to the outside of the cell membrane, where it can be recognized by a T lymphocyte, which is compatible with similar structures on the cell membrane of a B lymphocyte. If the B cell and T cell structures match, the T lymphocyte activates the B lymphocyte, which produces antibodies against the bits of antigen presented on its surface.

Most antigens are T-dependent, thus CD4+ T-helper cells required for maximal antibody production. When a B cell processes and presents an appropriate antigen to a T cell, the T helper cell secretes cytokines that activate the B cell. These cytokines trigger B cell proliferation and differentiation into plasma cells and the production of antibody. Suppressor T cells comprising CD8, on the other hand, reduce the production of antibody. Suppressor T cells are essential in the regulation of immune responses particularly as they relate to self antigens.

The term "Fc polypeptide" as used herein includes native and mutein forms of polypeptides made up of the Fc region of an antibody comprising any or all of the CH domains of the Fc region. Exemplary Fc polypeptides comprise an Fc polypeptide derived from a human IgG1 antibody. As one alternative, a fusion polypeptide is prepared using polypeptides derived from immunoglobulins operably linked to an antigenic polypeptide (e.g., pCons). Preparation of fusion polypeptides comprising certain heterologous polypeptides fused to various portions of antibody-derived polypeptides (including the Fc domain) have been described, e.g., by Ashkenazi et al. (PNAS USA 88:10535, 1991); Byrn et al. (Nature 344:677, 1990); and Hollenbaugh and Aruffo ("Construction of Immunoglobulin Fusion Polypeptides", in Current Protocols in Immunology, Suppl. 4, pages 10.19.1-.10.19.11, 1992). Tisch et al. (The Journal of Immunology, The Williams and Wilkins co. Baltimore, vol. 166, no. 3, January 2001, pp. 2122-2132) disclose a gene construct encoding a fusion protein consisting of a fragment of glutamic acid decarboxylase 65 (GAD65) linked to IgGFc and IL-4.

A fusion Fc construct or minigene comprise a polynucleotide encoding a polypeptide/Fc fusion polypeptide. Such a minigene can be inserted into an appropriate expression vector. Polypeptide/Fc fusion polypeptides are expressed in host cells transformed or transfected with the recombinant expression vector or recombinant polynucleotide encoding the fusion polypeptide, and allowed to assemble and be processed. One suitable Fc polypeptide, described in PCT application WO 93/10151,
is a single chain polypeptide extending from the N-terminal hinge region to the native C-terminus of the Fc region of a human IgG1 antibody. Another useful Fc polypeptide is the Fc mutein described in U.S. Pat. No. 5,457,035 and in Baum et al., (EMBO J. 13:3992, 1994).

The amino acid sequence of this mutein is identical to that of the native Fc sequence presented in WO 93/10151, except that amino acid 19 has been changed from Leu to Ala, amino acid 20 has been changed from Leu to Glu, and amino acid 22 has been changed from Gly to Ala. The above-described fusion polypeptides comprising Fc moieties offer the advantage of being processed by APC such that they are appropriate presented by the APCs. In other embodiments, the polypeptides of the invention can be substituted for the variable portion of an antibody heavy or light chain.
A "polynucleotide" generally refers to any polyribonucleotide (RNA) or polydeoxyribonucleotide (DNA), which may be unmodified or modified RNA or DNA. Polynucleotides include, without limitation, single-stranded and double-stranded DNA, DNA that is a mixture of single-stranded and double-stranded regions, single-stranded and double-stranded RNA, and RNA that is a mixture of single-stranded and double-stranded regions. Polynucleotides also include hybrid molecules comprising DNA and RNA that may be single-stranded or, more typically, double-stranded or a mixture of single-stranded and double-stranded regions. In addition, "polynucleotide" refers to triple-stranded regions comprising RNA or DNA or both RNA and DNA. Polynucleotides also include DNAs or RNAs containing one or more modified bases and DNAs or RNAs with backbones modified for stability or for other reasons. "Modified" bases include, for example, tritylated bases and unusual bases such as inosine. A variety of modifications may be made to DNA and RNA; thus, "polynucleotide" embraces chemically, enzymatically or metabolically modified forms of polynucleotides as typically found in nature, as well as the chemical forms of DNA and RNA characteristic of viruses and cells. Oligonucleotides are relatively short polynucleotides. Examples of polynucleotides used in the methods and compositions of the invention comprise a polynucleotide encoding a peptide with T-cell determinants in mammalian IgG, e.g. murine IgG or human IgG, particularly the consensus peptide pCons (FIEWNKLRFRQGLEW (SEQ ID NO:2), binding I-E^{d} and K^{d}). The artificial peptide pCons is based on an algorithm that defines the T cell stimulatory amino acid sequences from the V_{H} regions of murine IgG antibodies to DNA (Hahn et al., Arthritis & Rheumatism, John Wiley & Sons, Inc., US, vol. 44, no. 2, February 2001, pp. 432-441). In one aspect, the polynucleotide comprises the sequence 5'-TTTATCGAGTGGAATAAGCTGCGATTTCGTCAGGGCCTGGAGTGG-3' (SEQ ID NO: 1). Further, the invention describes a polynucleotide encoding a variant or functional fragment of the consensus peptide pCons, e.g. a variant wherein 1, 2 or 3 amino acids of the pCons sequence have been substituted by different amino acids or a functional fragment of pCons comprising 10, 11, 12, 13 or 14 consecutive amino acids of pCons or a variant thereof.

A "polypeptide" refers to any polypeptide comprising two or more amino acids joined to each other by peptide bonds or modified peptide bonds. "Polypeptide" refers to both short chains, commonly referred to as peptides, oligopeptides or oligomers, and to longer chains, generally referred to as proteins. Polypeptides may contain amino acids other than those normally encoded by a codon. Preferably, the polypeptides comprise a peptide with T-cell determinants in mammalian IgG, e.g. murine IgG or human IgG. An exemplary polypeptide comprises pCons (SEQ ID No: 2). Further, the invention describes a variant or functional fragment of the consensus peptide pCons, e.g. a variant wherein 1, 2 or 3 amino acids of the pCons sequence have been substituted by different amino acids. This polypeptide preferably has a length of at least 10, e.g. at least 15 amino acids and up to 100, e.g. up to 20 amino acids. In another aspect, a pCons polypeptide of SEQ ID NO:2 or a variant or fragment thereof may include one or more D-amino acids. D-amino acids (as opposed to L-amino acids) increase biostability and reduce degradation by enzymes.

Polypeptides include amino acid sequences modified either by natural processes, such as post-translational processing, or by chemical modification techniques that are well known in the art. Such modifications are well described in the literature and are known in the art. Modifications may occur anywhere in a polypeptide, including the peptide backbone, the amino acid side-chains and the amino or carboxyl termini. Such modifications may be present to the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, biotinylation, covalent attachment of flavin, covalent attachment of a heme moiety, covalent attachment of a nucleotide or nucleotide derivative, covalent attachment of a lipid or lipid derivative, covalent attachment of phosphotidylinositol, crosslinking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. The polypeptide useful in the methods and compositions of the invention comprises the pCons polypeptide set forth in SEQ ID NO: 2.

The invention provides pCons antigens that are immunoprotective by generating immune tolerance. Such antigens can be delivered in a number of ways to the host so as to stimulate a tolerogenic protective immune response. For example, the self-antigen (e.g., pCons) can be delivered as a fusion polypeptide. The fusion polypeptide comprises a self antigen linked to a heterologous polypeptide or small molecule. In particular, the present invention relates to a fusion polypeptide comprising a first domain comprising a self antigen which is a conserved sequence found in T cell determinants in the FR1/CDR1 region of V_{H} of human and murine IgG antibodies, wherein the self antigen is SEQ ID NO: 2, and a second domain comprising a heterologous polypeptide or small molecule. Typically the heterologous polypeptide or small molecule assist in the uptake, processing or delivery of the self antigen. Exemplary heterologous polypeptides includes Fc polypeptides, protein transduction domains (e.g., TAT), or other adjuvant polypeptide known in the art. Advantageously, the invention demonstrates that the pCons antigen delivered through B-cell somatic presentation provides improved tolerogenic response compared to direct injection.

The antigens of the present invention may be administered to a subject in need thereof, e.g. as a polynucleotide vaccine, a polypeptide vaccine or a live vaccine.

The invention provides a minigene comprising the self antigen pCons in operable association with a Fc polypeptide coding sequence. The minigene is used to deliver the antigen to the immune system of a subject.

Alternatively the antigens may be delivered by direct administration of the polypeptide to a subject in need thereof.

The antigens can, be delivered via an attenuated vector or genetically engineered cell comprising a minigene of the invention that results in presentation of the antigen via MHC class I and/or II. The term "attenuated," when used with respect to a bacteria or virus, means that the vector (e.g., bacteria or virus) has lost some or all of its ability to proliferate and/or cause disease or other adverse effect when the bacteria infects an organism. For example, an "attenuated" bacteria can be unable to replicate at all, or be limited to one or a few rounds of replication. Alternatively or additionally, an "attenuated" bacteria might have one or more mutations in a gene or genes that are involved in pathogenicity of the bacteria. Many genes, loci, or operons are known, mutations in which will result in an attenuated bacteria. Examples of attenuated bacteria used as live vaccines include S. typhi carrying a mutation in its galE or htrA gene, and V. cholerae carrying mutations in its ctxA gene. The delivery of pCons, for example, in a genetically engineered attenuated vector would result in the endocytosis and presentation of pCons in association with MHC such that T cells are appropriately suppressed as described above.

Microorganisms which are used to express the PCONs for use in immunoprotective compositions include, without limitation, Campylobacter sp., Yersinia sp., Helicobacter sp., Gastrospirillum sp., Bacteroides sp., Klebsiella sp., Lactobacillis sp., Streptococcus gordonii, Enterobacter sp., Salmonella sp., Shigella sp., Aeromonas sp., Vibrio sp., Clostridium sp., Enterococcus sp. and Escherichia coli (see e.g. U.S. Pat. Nos. 5,858,352, and 6,051,416, and Levine et al., in "New Generation Vaccines Second Edition" ed. Levine et al., Marcel Dekker, Inc. pp 351-361 (1997), Levine et al., in "New Generation Vaccines Second Edition" ed. Levine et al., Marcel Dekker, Inc. pp 437-446 (1997), Butterton et al., in "New Generation Vaccines Second Edition" ed. Levine et al., Marcel Dekker, Inc. pp 379-385 (1997) and Fennelly et al., in "New Generation Vaccines Second Edition" ed. Levine et al., Marcel Dekker, Inc. pp 363-377 (1997)). For example, Campylobacter jejuni, Campylobacter coli, Listeria monocytogenes, Yersinia enterocolitica, Yersinia pestis, Yersinia pseudotuberculosis, Escherichia coli, Shigella flexneri, Shigella sonnei, Shigella dysenteriae, Shigella boydii, Helicobacter pylori, Helicobacter felis, Gastrospirillum hominus, Vibrio cholerae, Vibrio parahaemolyticus, Vibrio vulnificus, Bacteroides fragilis, Clostridium difficile, Salmonella typhimurium, Salmonella typhi, Salmonella gallinarum, Salmonella pullorum, Salmonella choleraesuis, Salmonella enteritidis, Klebsiella pneumoniae, Enterobacter cloacae, and Enterococcus faecalis. Escherichia coli include but are not limited to entero-toxic, entero-hemorrhagic, entero-invasive, entero-pathogenic or other strains can be used in the invention.

Alternatively, or in addition to, a non-bacterial attenuated vector such as a replication-deficient viral vectors comprising a minigene of the invention may be used in the methods and compositions of the invention. Such viral vectors useful in the methods and compositions of the invention include, but are not limited to, Vaccinia, Avipox, Adenovirus, AAV, Vaccinia virus NYVAC, Modified vaccinia strain Ankara (MVA), Semliki Forest virus, Venezuelan equine encephalitis virus, and herpes viruses.

In yet a further aspect, autologous or allogenic antigen presenting cells (e.g., B cells) maybe genetically engineered using a suitable expression vector (including viral vectors) ex-vivo such that pCons is expressed within the cell in association with an Fc polypeptide to facilitate processing and presentation by APCs.

Examples of suitable viral vectors include herpes simplex viral vectors, vaccinia or alpha-virus vectors and retroviruses, including lentiviruses, adenoviruses and adeno-associated viruses. In one embodiment, these vectors are replication defective virus vectors. Gene transfer techniques using these viruses are known to those skilled in the art. Retrovirus vectors, for example, may be used to stably integrate the polynucleotide of the invention into the host genome, although such recombination may not be advisable. Replication-defective adenovirus vectors by contrast remain episomal and therefore allow transient expression.

In a specific embodiment, the adenovirus used as a live vector is a replication defective human or simian adenovirus. Typically these viruses contain an E1 deletion and may be grown on cell lines that are transformed with an E1 gene. Suitable Simian adenoviruses are, for example, viruses isolated from Chimpanzee. Examples of viruses suitable for use in the present invention include C68 (also known as Pan 9) (U.S. Pat. No. 6,083,716) and Pan 5, 6 and Pan 7 (WO 03/046124). Thus, these vectors can be manipulated to insert a heterologous polynucleotide coding for an antigen or minigene such that the product is expressed. The use formulation and manufacture of such recombinant adenoviral vectors is set forth in detail in WO 03/046142.

The invention provides an immunogenic composition and vaccine that uses a method to facilitate that delivers pCons immunogenic antigens and facilitates processing in a manner that provides an telerogenic antigenic presentation similar to natural processing. PCons antigens are delivered in one or more vectors capable of inducing presentation via Major Histocompatability Complex (MHC) Class II and Class I.

The attenuated delivery vector releases potentially immunoprotective antigens comprising an Fc polypeptide operably linked to a self antigen (e.g., pCons) into the host cell cytoplasm, after which they are processed and presented to the immune system. Such antigens are presented to the immune system via MHC class I molecules, resulting in the priming of CD8 T-cells including suppressor T cells.

the invention demonstrates that somatic IgG consensus peptide minigene transfer can reduce hypergammaglobulinemia and delay renal disease in recognized animal models of SLE (e.g., NZB/W F₁ mice). Sustained production of IgG causing Ig overload (a symptom of SLE) can be suppressed by CD8⁺ T cells. Of note, the suppression of CD4⁺ T-cell responses by minigene-induced CD8⁺CD28⁻ suppressors has interesting analogies with previous observations by Suciu-Foca and coworkers, where MHC class I-restricted Ag-specific CD8⁺CD28⁻ T cells were capable to suppress Ag-specific CD4⁺ T-cell proliferative responses via mechanisms that included anergy in their targets. Also, the finding of a protective effect of CD8⁺CD28⁻ T cells in SLE may be of interest in relation to the previous findings of a correlation between impaired function of CD8⁺ T suppressor cells and disease activity in SLE patients.

The mechanisms of protection induced by somatic minigene transfer of pCons differ from what was observed when administering pCons as soluble peptide to NZB/W F₁ mice. In those experiments, an expansion of Foxp3-expressing cells was observed that is not seen using pCons as minigene. The differences may be related to the fact that soluble peptides in vivo have accelerated catabolism as compared to the half-life of the encoded products of gene vaccination. Also, the long-lasting in vivo availability of pCons to APC and/or suppressor T cells provided by minigene vaccination lead to prolonged response or to a different handling for immune cells. For example, minigenes could cause availability of encoded genes within the endocytic pathway (where loading of MHC molecules occurs) - in a fashion similar to the handling of endogenous antigens (Ag) - rather than providing uptake of exogenous Ag as for soluble peptide. Whichever case may contribute to the protective effects of pCons minigene, the study expands the applicability of somatic B-cell vaccination to new possibilities. Somatic transfer of minigenes in as little as 70 B cells was shown to be effective in inducing protective T-cell immunity against influenza virus.

The invention demonstrates that somatic B-cell minigene transfer can induce protective tolerogenic responses in autoimmunity. The implications of this application indicate new possibilities for intervention with this strategy and suggest that induction of suppressor CD8⁺ T via this method can modulate immunoregulatory circuits and hypergammaglobulinemia.

A "vaccine" as used herein refers to a composition of matter comprising a molecule that, when administered to a subject, induces an immune reaction. In one aspect, the immune reaction is a suppression of T cell activation to a self antigen such as PCons. Vaccines can comprise polynucleotide molecules, polypeptide molecules, and carbohydrate molecules, as well as derivatives and combinations of each, such as glycoproteins, lipoproteins, carbohydrate-protein conjugates, fusions between two or more polypeptides or polynucleotides, and the like. A vaccine may further comprise a diluent, an adjuvant, a carrier, or combinations thereof, as would be readily understood by those in the art.

A vaccine may be comprised of separate components. As used herein, "separate components" refers to a situation wherein the vaccine comprises two discrete vaccines to be administered separately to a subject. In that sense, a vaccine comprised of separate components may be viewed as a kit or a package comprising separate vaccine components. For example, in the context of the invention, a package may comprise a first immunogenic composition comprising an attenuated bacterial vector and a second antigenic composition comprising an attenuated viral vector comprising the same or different self antigens.

A vaccine "induces" an immune reaction when the antigen or antigens present in the vaccine cause the vaccinated subject to mount or reduce an immune response to that antigen or antigens. The vaccinated subject will generate an immune response, as evidenced by activation of or reduction (suppression) of the immune system, which includes the production of vaccine antigen-specific B cells, and the suppression of CD4⁺ T cells with increased activity of CD8⁺CD28⁻ T cells. The resulting immune response may be measured by several methods including ELISPOT, ELISA, chromium release assays, intracellular cytokine staining, FACS analysis, and MHC tetramer staining (to identify peptide-specific cells). A skilled artisan may also use these methods to measure a primary immune response or a secondary immune response.

An "antigen" is a substance capable of generating an immune response in a subject exposed to the antigen. Antigens are usually polypeptides and are the focus of the host's immune response. An "epitope" or "antigenic determinant" is that part of an antigen to which T cells and antibodies specifically bind. An antigen may contain multiple epitopes. Antigens of the invention preferably comprise a conserved sequence found in T cell determinants in the FRI/CDR1 region of VH of human and murine IgG antibodies. An example of such an antigen includes pCons comprising SEQ ID NO:2.

In various aspects of the invention, the self antigen (e.g., pCons) is operably connected to an Fc polypeptide or other heterologous polypeptide by use of a linker. Where a minigene is used, the self antigen coding region and the Fc polypeptide can be separated by a linker coding region. Typically a linker will be a peptide linker moiety. The length of the linker moiety is chosen to optimize the biological activity of expression of a self antigen-Fc fusion polypeptide and can be determined empirically without undue experimentation. The linker moiety can be a peptide between about one and 30 amino acid residues in length, typically between about two and 15 amino acid residues. Exemplary linker moieties are --Gly-Gly-, GGGGS (SEQ ID NO:3), (GGGGS)ₙ (SEQ ID NO:4), GKSSGSGSESKS (SEQ ID NO:5), GSTSGSGKSSEGKG (SEQ ID NO:6), GSTSGSGKSSEGSGSTKG (SEQ ID NO:7), GSTSGSGKPGSGEGSTKG (SEQ ID NO:8), or EGKSSGSGSESKEF (SEQ ID NO:9). Linking moieties are described, for example, in Huston, J. S., et al., PNAS 85:5879 (1988), Whitlow, M., et al., Protein Engineering 6:989 (1993), and Newton, D. L., et al., Biochemistry 35:545 (1996). Other suitable peptide linkers are those described in U.S. Pat. Nos. 4,751,180 and 4,935,233. A DNA sequence encoding a desired peptide linker can be inserted between, and in the same reading frame as, DNA sequences of the invention, using any suitable conventional technique. For example, a chemically synthesized oligonucleotide encoding the linker can be ligated between a pCons polynucleotide sequence and an Fc polynucleotide sequence. In some embodiments, a fusion polypeptide can comprise from two to four self antigen (e.g., pCONs) and Fc polypeptide domains, separated by peptide linkers.

Each tolerogenic composition (vaccine) comprising a minigene of the invention expressed in an attenuated vector or autologous or allogenic immune cell is administered, e.g. subcutaneously, intramuscularly, intranasally, inhaled, or even orally to a mammalian subject. The composition/vaccine can be administered as part of a homologous or heterologous prime-boost strategy.

Each tolerogenic composition (vaccine) comprising a minigene of the invention expressed in an attenuated vector or autologous or allogenic immune cell or a fusion polypeptide comprising a pCons polypeptide is administered, e.g. subcutaneously, intramuscularly, intranasally, inhaled, or even orally to a mammalian subject. The composition/vaccine can be administered as part of a homologous or heterologous prime-boost strategy.

Attenuated vaccines can be administered directly to the mammal. The immunogenic compositions and vaccines obtained using the methods of the invention can be formulated as pharmaceutical compositions for administration in any suitable manner. One route of administration is oral. Other routes of administration include rectal, intrathecal, buccal (e.g., sublingual) inhalation, intranasal, and transdermal and the like (see e.g. U.S. Pat. No. 6,126,938). Although more than one route can be used to administer a particular composition, a particular route can often provide a more immediate and more effective reaction than another route (e.g., via ex-vivo cell engineering).

The immunoprotective compositions to be administered are provided in a pharmaceutically acceptable solution such as an aqueous solution, often a saline or buffered solution. There is a wide variety of suitable formulations of pharmaceutical compositions of the invention. See, e.g., Lieberman, Pharmaceutical Dosage Forms, Marcel Dekker, Vols. 1-3 (1998); Remington's Pharmaceutical Science, 17th ed., Mack Publishing Company, Easton, Pa. (1985) and similar publications. The compositions may also include an adjuvant.

Formulations suitable for oral administration can comprise (a) liquid solutions, such as an effective amount of the recombinant cell suspended in diluents, such as buffered water, saline or PEG 400; (b) capsules, sachets or tablets, each containing a predetermined amount of the immunogenic composition; (c) suspensions in an appropriate liquid; and (d) suitable emulsions. Tablet forms can include one or more of lactose, sucrose, mannitol, sorbitol, calcium phosphates, corn starch, potato starch, tragacanth, microcrystalline cellulose, acacia, gelatin, colloidal silicon dioxide, croscarmellose sodium, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise the active ingredient in a flavor, usually sucrose and acacia or tragacanth, as well as pastilles comprising the active ingredient in an inert base, such as gelatin and glycerin or sucrose and acacia emulsions, gels, and the like containing, in addition to the active ingredient, carriers known in the art. It is recognized that the attenuated vaccines or cellular preparations, when administered orally, must be protected from digestion. This is typically accomplished either by complexing the vaccines with a composition to render it resistant to acidic and enzymatic hydrolysis or by packaging the vaccines in an appropriately resistant carrier such as a liposome or enteric coated capsules. Means of protecting the attenuated bacteria, virus, or cellular preparation from digestion are well known in the art. The pharmaceutical compositions can be encapsulated, e.g., in liposomes, or in a formulation that provides for slow release of the active ingredient.

The attenuated vaccines, alone or in combination with other suitable components, can be made into aerosol formulations (e.g., they can be "nebulized") to be administered via inhalation. Aerosol formulations can be placed into pressurized acceptable propellants, such as dichlorodifluoromethane, propane, nitrogen, and the like.

The dose administered to a subject, in the context of the invention should be sufficient to effect a beneficial therapeutic and/or prophylactic response in the subject over time. The dose will be determined by the efficacy of the particular immuno-tolerogenic composition employed and the condition of the subject, as well as the body weight or vascular surface area of the subject to be treated. The size of the dose also will be determined by the existence, nature, and extent of any adverse side-effects that accompany the administration of a particular vaccine in a particular subject.

In determining the effective amount of the vaccine to be administered in the treatment or prophylaxis of an infection or other condition, the physician evaluates vaccine toxicities, progression of the disease, and the production of antibodies to the self-antigen or T helper cell responses, if any.

The compositions are administered to a subject that has or is at risk of acquiring an autoimmune disorder or disease (e.g., SLE) to at least prevent or at least partially arrest the development of the disease or disorder and its complications. An amount adequate to accomplish this is defined as a "therapeutically effective dose." Amounts effective for therapeutic use will depend on, e.g., the immuno-tolerogenic composition, the manner of administration, the weight and general state of health of the subject, and the judgment of the prescribing physician. Single or multiple doses of the compositions may be administered depending on the dosage and frequency required and tolerated by the subject, and route of administration. In addition, a booster may be administered in the same or different formulation.

In particular embodiments, a therapeutically effective dose of the immunoprotective composition is administered to a subject. Amounts of live attenuated bacteria or non-bacteria expressing the PCONs-Fc fusion polypeptide or other antigens generally range from about 5x10⁵ to 5x10¹¹ organisms per subject, and more commonly from about 5x10⁸ to 5x10⁹ organisms per subject.

The existence of an immune response to the first dose of the immunoprotective composition may be determined by known methods (e.g., by obtaining serum from the individual before and after the initial immunization, and demonstrating a change in the individual's immune status, for example an immunoprecipitation assay, or an ELISA, or a Western blot, or flow cytometric assay, or the like) prior to administering a subsequent dose. The existence of an immune response (e.g., a reduced immune response) to the first dose may also be assumed by waiting for a period of time after the first immunization that, based on previous experience, is a sufficient time for an immune response to have taken place.

The immunoprotective compositions are typically administered to an individual that is immunologically naive with respect to PCONs. Usually, 2-4 doses of an immunological composition of the invention may be sufficient, however additional doses may be required to achieve a high level of immunity. In general, administration to any individual should begin prior to the first sign of disease.

The toxicity and therapeutic efficacy of the composition provided by the invention are determined using standard pharmaceutical procedures in cell cultures or experimental animals. One can determine the ED₅₀ (the dose therapeutically effective in 50% of the population) using procedures presented herein and those otherwise known to those of skill in the art.

A minigene of the disclosure can be packaged for use in the clinical and research laboratories. For example, a minigene of the invention comprising a polynucleotide encoding a pCONs operably linked to an Fc polypeptide can be provided for use in generating an expression vector. Alternatively, the minigene may be provided in an expression vector. In yet another aspect, the minigene may be provided in a host vector for use in immunizing a subject. The immunogenic composition of the invention can be packaged in packs, dispenser devices, and kits for administering genetic vaccines to a mammal. For example, packs or dispenser devices that contain one or more unit dosage forms are provided. Typically, instructions for administration of the compounds will be provided with the packaging, along with a suitable indication on the label that the compound is suitable for treatment of an indicated condition.

The following specific examples are meant to be illustrative and nonlimiting. Those of skill in the art will recognize various modification and substitutions that can be made in the compositions and methods that follow. Such modification and substitutions do not depart from the invention and are encompassed herein.

### EXAMPLES

### Materials and Methods

Mice. (NZB x NZW)F₁ (NZB/W F₁) (H-2^{d/z}) mice were purchased from The Jackson Laboratory (Bar Harbor, ME) or bred at UCLA and treated in accordance with the Institutional guidelines. All experiments were performed on female mice.

Antigens. The consensus peptide pCons (FIEWNKLRFRQGLEW, binding I-E^{d} and K^{d}) and the negative control peptide pNeg (AIAWAKARARQGLEW) are synthetic peptides containing T cell determinants common to several different J558 V_{H} regions of anti-dsDNA IgG of NZB/W F₁ mice¹¹. Another control peptide, pHyHEL (VKQRPGHGLEWIGEI), derives from the CDR 1/framework 2 V_{H} region of a murine mAb to hen egg lysozyme (HEL) and also binds I-E^{d 10}. Peptides were synthesized by a microcrown method at Chiron (San Diego, CA), purified to single peak on HPLC, and analyzed by mass spectrometry for expected aminoacid content.

Minigene plasmid constructs. The pCMVscript vector (pCMV) (Stratagene, La Jolla, CA) contains the cytomegalovirus (CMV) promoter that drives the expression of cloned inserts in mammalian cells. Using pCMV as backbone, minigenes were inserted in the EcoRI site of the polylinker. pIg plasmid encodes C_{H2}-C_{H3} of IgG₁ cloned by PCR from human PBMC. The forward primer contains a start codon and the Xbal restriction site (underlined): 5-ATGTCTAGAGTTGAGCCCAAATCTTGTGAC-3', the reverse primer is specific for the 3' end of hIgG₁ (5'-CGGCCGTCGCACTCATTTACC-3'). PCR cycling conditions were: 95°C for 2 min, followed by 94°C for 30 sec, 57°C for 30 sec and 72°C for 45 sec for 30 cycles, then 72°C for 10 min. pIgCons and pIgNeg plasmids encode both pIg and pCons or pNeg peptides, respectively (Figure 1a). Oligonucleotides for pCons were: 
5'-
CTAGATTTATCGAGTGGAATAAGCTGCGATTTCGTCAGGGCCTGGAGTG
GA-3' and
 
5'-
CTAGTCCACTCCAGGCCCTGACGAAATCGCAGCTTATTCCACTCGATAA
AT-3',
 for pNeg: 
5'-
CTAGAGCTATCGCTTGGGCTAAAGCTCGCGCTAGACAAGGTTTAGAGT
 GGA-3' and

5’-
CTAGTCCACTCTAAACCTTGTCTAGCGCGAGCTTTAGCCCAAGCGATAG
CT-3’.

For each plasmid, forward and reverse oligonucleotides were annealed and inserted in frame at the 5' end of the human IgG₁ sequence within the Xbal sites. Plasmid DNA was purified from transformed E. Coli using endo-free Maxi-prep kits (Qiagen, Valencia, CA). Total RNA extraction and cDNA synthesis for RT-PCR confirming expression of mRNA transcripts were performed following standard procedures. Total cellular RNA was extracted with TRIzol reagent (Invitrogen Life Technologies, Carlsbad, CA) from 3 x 10⁶ cells. RT-PCR was performed using the Invitrogen Superscript One-Step RT-PCR with Platinum Taq kit on a Hybrid PCR Express thermocycler (Milford, MA). Amplification was performed with the common reverse primer 5'- GTCACAAGATTTGGGCTCAAC-3' and the following forward primers: for IgG₁, 5'-ATGTCTAGAGTTGAGCCCAAATCTTGTGAC-3'; for pCons, 5'-ATGTCTAGATTTATCGAGTGG-3'; for pNeg, 5'-ATGTCTAGAGCTATCGCTTG-3'.

The PCR conditions used were: 95°C for 2 min, followed by 94°C for 30 sec, 57°C for 30 sec, 72°C for 45 sec for 30 cycles, and 72°C for 10 min. The housekeeping β-actin gene was amplified in parallel using the same PCR conditions with the primers: 5'-GCTCGTCGTCGACAACGGCTC-3' and 5'-CAAACATGATCTGGGTCATCTTCTC-3'. Sequence analyses were done via automated sequencing on an ABI 3100 machine using Big Dye Terminator (Applied Biosystem, Foster City, CA).

In selected experiments, eukaryotic COS-7 cells (ATCC, Manassas, VA) were transfected with the plasmids using Fugene 6 (Roche, Indianapolis, IN), in accordance with the manufacturer's instructions. Resolution of protein lysates was done by western blot using a goat anti-human IgG₁-HRP conjugate (Sigma, Saint Louis, MO).

Somatic B-cell minigene transfer. Somatic B-cell minigene transfer has been described in detail elsewhere. Briefly, single spleen cell suspensions were prepared from mice in aseptic conditions and B cells sorted for enrichment (≥ 96%) using anti-CD19 magnetic beads (Miltenyi Biotec, Auburn, CA) on a VarioMACS separator (Miltenyi Biotec). 4 x 10⁶ purified B cells were resuspended in 200 µl of PBS containing Ca²⁺ and Mg²⁺ and incubated with 25 µg of plasmid for 1 h at 37°C. Cells were then diluted in complete medium (RPMI 1640 supplemented with 10% FCS, 10mM Hepes, 200mM glutamine, 100mM sodium pyruvate and non essential amino acids) and incubated overnight at 37°C in 5% CO₂. The persistence of the expression of minigenes in transfected cells lasted up to a month (37), and efficiency of transfection prior to transfer into mice was always evaluated by fluorescence-activated cell sorting via surface staining with FITC-conjugated mAb to CD19 (BD Biosciences, San Diego, CA) coupled to intracellular staining with FITC-conjugated anti-human IgG₁ mAb (Sigma). Intracellular staining was done using the BD Cytofix/Cytoperm kit, following the manufacturer's instructions. B cells transfected as described above were washed in PBS and diluted in 200 µl of PBS for transfer into mice. The number of minigene-expressing lymphocytes was estimated by fluorescence-activated cell sorting prior to transfer of 6x10⁵ transfected B cells into each mouse. The plasmids used for somatic-B cell minigene transfer and treatment of the mice were pIg, pIgCons, pIgNeg, and pCMV. A control group of mice received only PBS.

Monitoring of mice. Proteinuria was assessed in all groups of mice pre- and post-treatment, at weekly intervals, using Albustix strips (Bayer, Elkhart, IN).

Histology. Kidney sections (4-pm-thick) were stained hematoxylin and eosin (H/E) following standard procedures. Pathology scoring included the glomerular activity score (GAS) and tubulointerstitial activity score (TIAS) and was done in a blinded fashion on a 0 to 3 scale where 0 = absence of lesions; 1 = lesions in <30% of glomeruli; 2 = lesions between 30% to 60%; 3 = lesions >60% of glomeruli. The GAS includes glomerular proliferation, karyorrhexis, fibrinoid necrosis, inflammatory cells, cellular crescents and hyaline deposits. The TIAS includes interstitial inflammation, tubular cell necrosis and/or flattening, and epithelial cells or macrophages in tubular lumen. The raw scores were averaged to obtain a mean score for each individual feature and the mean scores were then summed to obtain an average score to obtain a composite kidney biopsy score. For immunofluorescence studies, sections were fixed in cold acetone for 10 minutes, washed and blocked with 2% bovine serum albumin (BSA) for 1 hour prior to addition of rabbit anti-mouse IgG or rabbit anti-human IgG (Sigma) followed by FITC-conjugated anti-rabbit antibodies (BD Biosciences) and counterstaining with H/E.

T cell proliferation assays. Splenocytes (recovered after red blood cell lysis) were seeded in triplicate wells at 2-5x10⁵ cells/well in a volume of 200 µl of HL-1 medium (Cambrex, Rockland, ME) in the presence of peptides (20 µg/ml) and /or 100U of recombinant IL-2 (R&D Systems, Minneapolis, MN). Cultures with medium alone or containing concanavalin A were used as negative and positive controls, respectively. Cells were maintained at 37°C in 5% CO₂ for 3 days and pulsed with 1 µCi of [³H]-Thymidine ([³H]-Thy) for the last 12-18 h; DNA incorporation of [³H]-Thy was assessed by liquid scintillation counting in an automated counter (Beckman Coulter, Fullerton, CA). Results are expressed as mean stimulation index ± SD of triplicates of groups of 6 to 8 mice each.

ELISA. Sera were collected from NZB/W F₁ mice before and after minigene treatment and stored at -80°C until experimental use. Ab titers and total serum levels of IgG, IgG₁ and IgG₂ₐ, were tested using commercial ELISA kits from BD Biosciences and R&D Systems, following the manufacturers' instructions.

Flow cytometry. After wash and Fc-gammaR blocking, Ab to surface markers or control isotype-matched fluorochrome-labeled Ab were added for 20 min at 4° C in PBS/2% FCS. For surface staining, the following fluorochrome-labeled mAb were used: anti-CD3, anti-CD4, anti-CD8, anti-CD25, anti-CD28, anti-CD19, anti-NK1.1, anti-CD44, anti-CD62L, anti-CD45RB, anti-CD69. Intracellular staining was performed subsequently with labeled anti-Foxp3 or anti-TGF-beta mAb using the manufacturers' instructions. All mAb were from BD Biosciences except anti-Foxp3 mAb (eBiosciences, San Diego, CA).

Statistical analyses. Differences between groups of continuous outcomes were compared using the Student's t-test. Differences between groups continuous outcomes evaluated at baseline and discrete follow-up time points were evaluated using paired t-tests. Survival between groups was modeled using Kaplan-Meier analysis. All analyses were conducted using Prism 4 software (GraphPad, San Diego). Values of P<0.05 were considered significant.

Construction and expression of minigenes. Premorbid NZB/W F₁ mice underwent somatic minigene transfer of plasmid encoding human IgG₁ (hIgG) (pIg plasmid) (Figure 1a). This approach allowed discrimination between minigene-derived IgG and endogenous mouse IgG. Additional constructs used in the study included: i) pCMV plasmid, a negative control empty plasmid; ii) pIgNeg, a plasmid which encodes hIgG₁ together with pNeg - a peptide that binds MHC class II but has no effect on T-cell activation or disease in NZB/W F₁ mice; and iii) pIgCons, a plasmid which encodes hIgG together with pCons Ig consensus peptide - pCons is a peptide that protects NZB/W F₁ mice from SLE. Validation of mRNA transcripts was done by RT-PCR on COS-7 cells transfected with pIgCons or pIgNeg or pIg plasmids (Figure 1b) and Ig expression analyzed by western blot on cell lysates using rabbit anti-hIgG₁ mAb (Figure 1c). Finally, a pCons-specific T cell line proliferated in responses to B cells transfected with pIgCons but not to B cells that had been transfected with pIg (Figure 1 d) or with the other control plasmids.

Somatic B-cell minigene transfer with pIgCons protects NZB/W F₁ mice from accelerated renal disease. Twenty to twenty-two week-old prenephritic female NZB/W F₁ mice with comparable low levels of anti-DNA Ig received each 6x10⁵ B cells transfected with pIg (n=19 mice) or pIgCons (n=19 mice) i.v. once. Control mice received similar numbers of B cells transfected with either pIgNeg (n=11) or pCMV (n=6), or received PBS only (n=8). Proteinuria was measured before beginning of treatment (no mouse was proteinuric when treatment was initiated) and monitored at weekly intervals thereafter. For measurement of Ig titers, sera were collected from peripheral blood before treatment and every other week after treatment for 30 weeks.

Mice that received pIg developed accelerated proteinuria as compared to control mice that had received either the empty plasmid pCMV or PBS (Figure 2a). No significant differences were observed among the pCMV- and PBS-treated control mice, suggesting that the plasmid per se did not influence renal disease in the treated animals. Significantly, mice treated with pIgCons had considerably lower levels of proteinuria at both 5 and 10 weeks after treatment in comparison with mice treated with pIg (Figure 2a). Protection from pIg-induced accelerated renal disease was specifically associated with pCons, since mice that had received pIgNeg had accelerated development of proteinuria similar to that of pIg-treated mice (Figure 2a).

Survival of mice and renal histopathology. The effects of somatic minigene transfer on proteinuria were associated with different survival of treated animals. The deleterious effects of hypergammaglobulinemia on disease prognosis were reflected by accelerated mortality of pIg-treated mice as compared to pIgCons-treated mice (Figure 2b). The pIgNeg-treated animals had a similar low rate of survival as pIg-treated mice, suggesting that only pCons exerted protective effects on the Ig accelerated disease that resulted in increased survival of the mice. Moreover, the plasmid per se did not influence mice survival because pCMV-treated mice had a rate of survival similar to that of PBS-treated controls (Figure 2b).

Renal pathology was analyzed in the different groups of mice (Figure 3). The architecture of the kidneys was preserved in mice treated with pIgCons as compared to pig and pNeg control mice (Figure 3a). Since the renal architecture of pCMV mice that had received the empty vector was relatively preserved, the plasmid per se did not influence renal pathology. Importantly, precipitation of hlg was observed in the glomeruli of pIg and pIgNeg mice but not in pIgCons mice (Figure 3b), and precipitation of mlg was observed in controls but not in the pIgCons treated mice (Figure 3c). Also, the glomerular and tubular activity scores were lower in the plgCons-treated mice than in pIg- and pIgNeg-treated control mice (Figure 3d).

Ig expression in treated animals. The finding that mice that had received pIg and pIgNeg had accelerated renal disease as compared to pIgCons-treated mice or to control mice treated with PBS or pCMV suggested that minigene expression of Ig had contributed to the renal disease unless pCons was expressed concomitantly. The protecting effect mediated by pCons could be related to the blockage of elevated production of Ig derived from the plasmid or to a blockage of endogenous Ig production. To discriminate between these two possibilities, the serum titers of minigene-derived hIgG were analyzed in the different groups of mice at five and ten weeks after treatment (Figure 4). It was found that the protective effects of pCons were not related to a differential expression of hIgG in the different groups of mice because similar levels of hIgG were detected in the sera of mice that had received pIg, pIgCons and pIgNeg (Figure 5a). As a control, hIgG were not detectable in the sera of mice that had not received minigenes encoding IgG but that had either received the empty plasmid or PBS (Figure 4a). These data indicated that plasmid-derived expression of Ig was comparable in the different groups of mice and that the protective effects observed in pIgCons-treated mice had to be ascribed to pCons. Since gene therapy induces Ab to the encoded gene product¹²⁻¹⁴ and an anti-hlgG response could have influenced the titer of circulating hIgG, the serum concentration of anti-hIgG Ab was analyzed in the different groups of mice. Similar (low) levels of anti-hlgG Ab were found in mice receiving plg, plgCons and plgNeg, and absence in the pCMV- and PBS-treated control mice. Importantly, however, the analysis of the serum levels of murine IgG after treatment indicated only the mice treated with plgCons had reduced titers of IgG at both five and ten weeks post-treatment (Figure 4b), indicating an association between pCons and reduced endogenous IgG production. All other conditions did not affect the serum concentration of circulating mouse IgG.

Cellular immune responses induced by plgCons. T-cell responsiveness was compared among the groups of mice treated with the different minigenes. Ag-specific lymphocyte proliferation was measured at 4 weeks and 8 weeks post-treatment in the absence or in the presence of rIL-2. As shown in Figure 6, no significant proliferation was observed in any group of mice to the Ag of the respective minigene product. However, addition of exogenous IL-2 to the cultures reversed hyporesponsiveness to stimulation with pCons in the plgCons-treated mice and not in plgNeg-treated mice or in the other controls, both at 4 and 8 weeks post-treatment (Figure 5). These data indicated that only plgCons-treated animals had T cells that were hyporesponsive to antigenic stimulation. To better understand the implications of this observation, flow cytometry was used to determine whether administration of plgCons influenced the number of selected splenic immune cell subsets including T, B, and NK cells. No significant changes were observed in the percentage numbers of B cells or NK cells after minigene treatment for as long as two months of monitoring after treatment. For T cells, expansion of CD8⁺ T cells was observed in the plgCons group as compared to the control groups (Figure 6a). For CD4⁺ T cells, there was no difference in the phenotype and/or expression of CD25, CD44, CD62L, CD45RB or CD69. Instead, the expansion of the CD8⁺ T cell compartment after treatment with plgCons associated with increased number of CD8⁺CD28⁻ T cells (Figure 6b-c), which is a phenotype that has previously been associated with T-cell suppression¹⁷⁻²⁰. Of note, the expanded CD8⁺CD28⁻ T cells in plgCons-treated mice expressed intracellular TGF-beta, which was not expressed in CD8⁺CD28⁻ T cells from plgNeg-treated mice or controls (Figure 6d). These phenotypic differences in plgCons mice vs controls were present as soon as 2 weeks after treatment. (Figure 6) and became more pronounced by 4 weeks after treatment. Of note, sorted CD8⁺CD28⁻ T cells from plgCons-treated animals - but not from the other groups of mice - inhibited the proliferation of stimulated CD4⁺ T cells (Figure 7a). The suppressive effects were maintained in transwell experiments and blocked by the presence in culture of anti-TGF-beta Ab, indicating that the suppression mediated by CD8⁺CD28⁻ T cells did not require cell contact and depended in part on TGF-beta. To test whether plgCons-derived CD8⁺ suppressors could delay the development of renal disease in vivo, adoptive transfer experiments were performed. It was found that the transfer of CD8⁺CD28⁻ T cells from plgCons-treated mice into NZB/W F₁ mice with hypergammaglobulinemia delayed the development of proteinuria in recipient animals, compared to mice receiving CD8⁺CD28⁻ T cells from plgNeg-treated controls (Figure 7b).

## Claims

1. A polynucleotide encoding a fusion polypeptide, wherein the fusion polypeptide comprises a self antigen operably linked to an Fc region of an antibody, wherein the self antigen is pCons SEQ ID NO:2.

2. The polynucleotide of claim 1, wherein the Fc region of an antibody comprises an IgG1 CH domain.

3. An expression vector comprising the polynucleotide of any one of claims 1-2.

4. The polynucleotide of claim 1 for use in prevention or treatment of systemic lupus erythematosus by inducing tolerogenic immunity in a subject, wherein the polynucleotide is for expression in the subject.

5. The polynucleotide for use according to claim 4, wherein the polynucleotide is to be transformed or transfected into an immune cell of the subject.

6. The polynucleotide for use according to claim 5, wherein the immune cell is a B-cell.

7. The polynucleotide for use according to claims 5 or 6, wherein the cell is to be transformed *ex vivo.*

8. A fusion polypeptide comprising:
a first domain comprising a self antigen which is a conserved sequence found in T cell determinants in the FR1/CDR1 region of V_{H} of human and murine IgG antibodies, wherein the self antigen is SEQ ID NO:2 ; and
a second domain comprising a heterologous polypeptide or small molecule.

9. The fusion polypeptide of claim 8, wherein the heterologous polypeptide comprises an Fc region of an antibody or an adjuvant polypeptide.

10. The fusion polypeptide of claim 8, wherein the small molecule comprises an adjuvant molecule.

11. A pharmaceutical composition comprising the fusion polypeptide of claim 8.

12. A fusion polypeptide of claim 8 for use in the treatment of an autoimmune disorder for repressing an immune response to said self antigen, particularly a self antigen comprising SEQ ID NO:2, wherein the autoimmune disorder is systemic lupus erythematosus (SLE).

## Patentansprüche

1. Polynukleotid, das für ein Fusionspolypeptid kodiert, wobei das Fusionspolypeptid ein Selbstantigen umfasst, das operativ mit einer Fc-Region eines Antikörpers verbunden ist, wobei das Selbstantigen pCons SEQ ID NO:2 ist.

2. Polynukleotid nach Anspruch 1, wobei die Fc-Region eines Antikörpers eine IgG1 CH-Domäne umfasst.

3. Expressionsvektor umfassend das Polynukleotid nach einem der Ansprüche 1-2.

4. Polynukleotid nach Anspruch 1 zur Verwendung in der Prävention oder Behandlung von systemischem Lupus erythematodes durch Induzieren tolerogener Immunität in einem Individuum, wobei das Polynukleotid zur Expression in dem Individuum ist.

5. Polynukleotid zur Verwendung nach Anspruch 4, wobei das Polynukleotid in eine Immunzelle des Individuums transformiert oder transfiziert werden soll.

6. Polynukleotid zur Verwendung nach Anspruch 5, wobei die Immunzelle eine B-Zelle ist.

7. Polynukleotid zur Verwendung nach Anspruch 5 oder 6, wobei die Zelle *ex vivo* transformiert werden soll.

8. Fusionspolypeptid umfassend:
eine erste Domäne umfassend ein Selbstantigen, die eine in T-Zell-Determinanten in der FR1/CDR1-Region von V_{H} von humanen und Maus-IgG-Antikörpern gefundene konservierte Sequenz ist, wobei das Selbstantigen SEQ ID NO:2 ist; und
eine zweite Domäne umfassend ein heterologes Polypeptid oder eine niedermolekulare Verbindung.

9. Fusionspolypeptid nach Anspruch 8, wobei das heterologe Polypeptid eine Fc-Region eines Antikörpers oder ein Adjuvans-Polypeptid umfasst.

10. Fusionspolypeptid nach Anspruch 8, wobei die niedermolekulare Verbindung ein Adjuvans-Molekül umfasst.

11. Pharmazeutische Zusammensetzung umfassend das Fusionspolypeptid nach Anspruch 8.

12. Fusionspolypeptid nach Anspruch 8 zur Verwendung in der Behandlung einer Autoimmunerkrankung zur Unterdrückung einer Immunantwort gegen das Selbstantigen, insbesondere ein Selbstantigen umfassend SEQ ID NO:2, wobei die Autoimmunerkrankung systemischer Lupus erythematodes (SLE) ist.

## Revendications

1. Polynucléotide codant pour un polypeptide de fusion, où le polypeptide de fusion comprend un auto-antigène en liaison fonctionnelle avec une région Fc d'un anticorps, où l'auto-antigène est pCons de SEQ ID NO: 2.

2. Polynucléotide selon la revendication 1, dans lequel la région Fc d'un anticorps comprend un domaine CH d'IgG1.

3. Vecteur d'expression comprenant le polynucléotide selon l'une quelconque des revendications 1 à 2.

4. Polynucléotide selon la revendication 1 pour utilisation dans la prévention ou le traitement du lupus érythémateux systémique en induisant une immunité tolérogène chez un sujet, où le polynucléotide est pour une expression chez le sujet.

5. Polynucléotide pour utilisation selon la revendication 4, où le polynucléotide doit être transformé ou transfecté dans une cellule immunitaire du sujet.

6. Polynucléotide pour utilisation selon la revendication 5, où la cellule immunitaire est une cellule B.

7. Polynucléotide pour utilisation selon les revendications 5 ou la revendication 6, où la cellule doit être transformée *ex vivo.*

8. Polypeptide de fusion comprenant :
un premier domaine comprenant un auto-antigène qui est une séquence conservée observée dans des déterminants de cellules T dans la région FR1/CDR1 de V_{H} d'anticorps IgG humains et murins, où l'auto-antigène est SEQ ID NO: 2 ; et un second domaine comprenant un polypeptide hétérologue ou une petite molécule.

9. Polypeptide de fusion selon la revendication 8, dans lequel le polypeptide hétérologue comprend une région Fc d'un anticorps ou un polypeptide adjuvant.

10. Polypeptide de fusion selon la revendication 8, dans lequel la petite molécule comprend une molécule adjuvante.

11. Composition pharmaceutique comprenant le polypeptide de fusion selon la revendication 8.

12. Polypeptide de fusion selon la revendication 8 pour utilisation dans le traitement d'un trouble auto-immun pour réprimer une réponse immunitaire contre ledit auto-antigène, particulièrement un auto-antigène comprenant SEQ ID NO: 2, où le trouble auto-immun est le lupus érythémateux systémique (LES).
